(19) Europäisches Patentamt
European Patent Office
Office européen des brevets

(11) Veröffentlichungsnummer: **0 073 411**
**A1**

(12) # EUROPÄISCHE PATENTANMELDUNG

(21) Anmeldenummer: **82107506.6**

(22) Anmeldetag: **18.08.82**

(51) Int. Cl.³: **C 07 D 249/12**, A 01 N 43/64

(30) Priorität: **24.08.81 DE 3133405**

(43) Veröffentlichungstag der Anmeldung: **09.03.83**
**Patentblatt 83/10**

(84) Benannte Vertragsstaaten: **AT BE CH DE FR GB IT LI LU NL SE**

(71) Anmelder: **BASF Aktiengesellschaft,**
**Carl-Bosch-Strasse 38, D-6700 Ludwigshafen (DE)**

(72) Erfinder: **Rentzea, Costin, Dr., Neuenheimer Landstrasse 72, D-6900 Heidelberg (DE)**
Erfinder: **Thym, Sabine, Dr., Hasenhain 20, D-6901 Dossenheim (DE)**
Erfinder: **Ammermann, Eberhard, Dr., Sachsenstrasse 3, D-6700 Ludwigshafen (DE)**
Erfinder: **Pommer, Ernst-Heinrich, Dr., Berliner Platz 7, D-6703 Limburgerhof (DE)**

(54) **1-(Trihalogenmethyl-sulfenyl)-4-aryl-1,2,4-triazolidin-5-one und diese enthaltende Fungizide.**

(57) 1-(Trihalogenmethyl-sulfenyl)-4-aryl-1,2,4-triazolidin-5-on der allgemeinen Formel

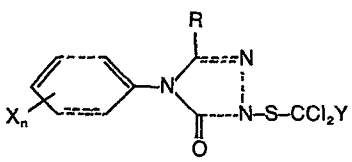

(I)

in der

R einen gegebenenfalls substituierten Alkylrest oder einen Alkenylrest oder einen Cacloalkylrest bedeutet,

X Wasserstoff, Halogen, eine gegebenenfalls substituierte Alkyl- oder Alkoxygruppe, Alkenyl, Cyan, Nitro, Phenyl oder Phenoxy bedeutet, wobei der Phenyl- oder der Phenoxyrest substituiert sein kann,

n eine ganze Zahl von 1 bis 5 ist und

Y Fluor oder Chlor bedeutet und diese enthaltende Fungizide.

EP 0 073 411 A1

# 1-(Trihalogenmethyl-sulfenyl)-4-aryl-1,2,4-triazolidin-5-one und diese enthaltende Fungizide

Die vorliegende Erfindung betrifft neue wertvolle 1-(Trihalogenmethyl-sulfenyl)-4-aryl-1,2,4-triazolidin-5-one mit fungizider Wirkung sowie deren Verwendung zur Bekämpfung phytopathogener Pilze und Fungizide, die diese Verbindungen enthalten.

Es ist bekannt, N-Trichlormethylthio-tetrahydrophthalimid zur Bekämpfung von phytopathogenen Pilzen zu verwenden (Chemical Week 1972, Juni 21, Seite 63). Seine Wirkung ist jedoch gegen andere Phycomyceten, z.B. Phytophthora infestans bei Tomaten oder Kartoffeln nicht befriedigend. Zum Materialschutz oder zum Schutz von Holz gegen holzverfärbende Pilze genügt seine Wirkung nicht.

Es ist ferner bekannt, 1-Halohydrocarbylthio-3-hydro-carbylthio-4-substituierte-1,2,4-triazolidin-5-one als Fungizide zu verwenden (US-PS 4 098 896). Dieser Veröffentlichung entspricht die Verbindung 1-Trichlormethylthio-3-methylthio-4-p-methoxy-m-chlorphenyl-1,2,4-triazolidin-5-on.

Es wurde nun gefunden, daß die neuen 1-(Trihalogenmethyl-sulfenyl)-4-aryl-1,2,4-triazolidin-5-one der allgemeinen Formel

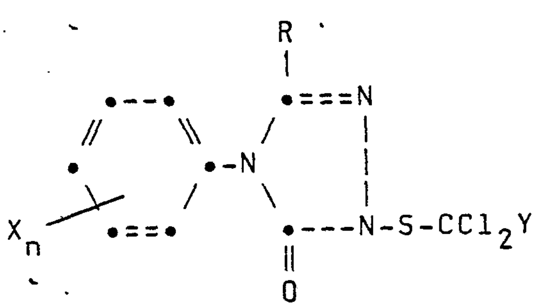

(I)

Sws/P

in der
R einen gegebenenfalls durch Methoxyl oder Ethoxyl substituierten Alkylrest mit 1 bis 6 C-Atomen, oder einen Alkenylrest mit 2 bis 6 C-Atomen oder einen gegebenenfalls durch Methoxyl substituierten Cycloalkylrest mit 3 bis 7 C-Atomen bedeutet,
X Wasserstoff, Halogen (z.B. Fluor, Chlor, Brom oder Jod), eine gegebenenfalls durch Fluor, Chlor oder Brom substituierten Alkyl- oder Alkoxygruppe mit 1 bis 5 C-Atomen, Alkenyl mit 2 bis 4 C-Atomen, Cyan, Nitro, Phenyl oder Phenoxy bedeutet, wobei der Phenyl- oder der Phenoxyrest durch Fluor, Chlor, Brom oder Alkyl mit 1 bis 4 C-Atomen substituiert sein kann und
n eine ganze Zahl von 1 bis 5 ist und
Y Fluor oder Chlor bedeutet, eine starke fungizide Wirkung haben. Die neuen Stoffe haben ein breites Wirkungsspektrum und können angewandt werden vor allem gegen Phycomyceten und Fungi imperfecti aber auch gegen Ascomyceten und Basidiomyceten. Die neuen Verbindungen sind beispielsweise geeignet zur Anwendung im Pflanzenschutz zur Bekämpfung phytopathogener Pilze. Dabei schädigen sie Kulturpflanzen in den zur Bekämpfung von Pilzen notwendigen Konzentrationen nicht. Die neuen 1-(Trihalogenmethyl-sulfenyl)-4-aryl-1,2,4-triazolidin-5-one eignen sich ferner hervorragend zum Materialschutz und zum Schutz von Holz gegen Pilzarten wie Sclerophoma und Pullularia.

Weiterhin wurde gefunden, daß man die Verbindungen der allgemeinen Formel (I) erhält, wenn man ein 4-Aryl-1,2,4-triazolidin-5-on der Formel (II)

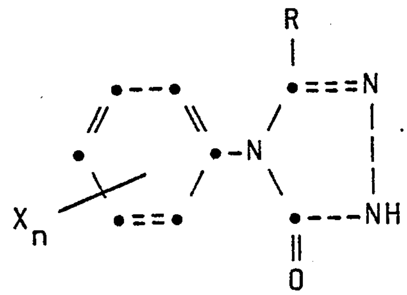

(II)

mit Sulfenylchloriden der Formel $YCCl_2-S-Cl$ (III), wobei R, X, Y und n die oben angegebenen Bedeutungen haben, gegebenenfalls in Gegenwart eines Säurebindemittels und gegebenenfalls in Gegenwart eines Lösungs- bzw. Verdünnungsmittels umsetzt. Die Umsetzungen werden zweckmäßig in gegenüber den Reaktionsteilnehmern inerten Lösungs- oder Verdünnungsmitteln, z.B. Wasser, Toluol, Xylol, Diethylether, Tetrahydrofuran, Dioxan, Dimethoxyethan, Aceton, Methylethylketon, Essigsäureester, Methylenchlorid, Chloroform, Dichlorethan oder Chlorbenzol ein- oder zweiphasig durchgeführt.

Als säurebindende Mittel kommen z.B. anorganische Basen wie Hydroxide oder Carbonate von Alkali- und Erdalkalimetallen (z.B. NaOH, $NaHCO_3$, $KHCO_3$, $K_2CO_3$, $CaCO_3$, $BaCO_3$) und vor allem tertiäre Amine, wie Triethylamin, N,N-Dimethyl-cyclohexylamin, N,N-Dimethylanilin oder Pyridin in Frage.

Die Umsetzungen erfolgen beispielsweise bei Temperaturen zwischen -30 und +100°C, vorzugsweise zwischen -10 und +25°C und bei normalem Druck.

Die neuen Verbindungen der allgemeinen Formel (I), wobei Y Fluor bedeutet, erhält man ferner, wenn man ein 1,2,4-Triazolidin-5-on der Formel Ia

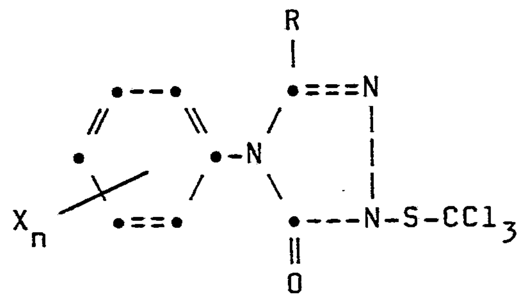

(Ia)

in der
R, X und n die oben angegebenen Bedeutungen haben, mit wasserfreier Fluorwasserstoffsäure umsetzt, um in der Trichlormethylthio-Seitenkette ein Chloratom gegen Fluor auszutauschen. Die Umsetzung mit einer Verbindung der Formel Ia kann in einem Überschuß von Fluorwasserstoffsäure als Verdünnungsmittel bei Temperaturen zwischen -50 und +80°C, vorzugsweise zwischen -10 und +25°C unter normalen oder erhöhtem Druck durchgeführt werden.

X bedeutet vorzugsweise Wasserstoff, Fluor, Chlor, Brom, Methyl, Ethyl, Isopropyl, tert.-Butyl, Trifluormethyl, Nitro, Methoxy, Ethoxy, Tetrafluorethoxy, Phenoxy und Phenyl, wobei n vorzugsweise für 1, 2, 3 oder 4 steht.

R steht bevorzugt für Methyl, Ethyl, n-Propyl, Isopropyl, n-Butyl, Isobutyl, sek.-Butyl, tert.-Butyl, n-Pentyl, Isopentyl, Methoxymethyl, 2-Methoxyethyl, 2-Ethoxyethyl, Cyclopropyl, Cyclopentyl, Cyclohexyl und 4-Methoxycyclohexyl.

Die als Ausgangsstoffe zu verwendenden 4-Aryl-1,2,4-triazolidin-5-one sind durch die Formel (II) allgemein definiert.

Die Ausgangsstoffe der Formel (II) sind bekannt (vgl. H. Gehlen u. W. Schade, Liebigs Ann. chem., 675, 180 (1964) und M. Pesson u.S. Dupin, Bull.Soc.chim. France, 250 (1962)). Noch nicht bekannte Ausgangsstoffe der Formel (II) können nach dem bekannten Verfahren erhalten werden, indem man 1-Aryl-4-formyl-semicarbazide in Gegenwart von Alkalihydroxiden cyclisiert.

Die Ausgangsstoffe der Formel (II) die für die Herstellung der neuen Verbindungen der Formel (I) verwendet wurden, sind in der Tabelle 1 im einzelnen beschrieben.

Die weiterhin für die Herstellung der neuen Stoffe erforderlichen 1-Trichlormethyl-sulfenyl-4-aryl-1,2,4-triazolidin-3-one sind durch die Formel (Ia) allgemein definiert. Sie können nach prinzipiell bekannten und laboratoriumsüblichen Verfahren hergestellt werden. Angaben dazu finden sich bei den Herstellungsbeispielen.

Die schließlich für die Herstellung der neuen Stoffe erforderlichen Trihalogenmethyl-sulfenyl-chloride der Formel III sind allgemein bekannt.

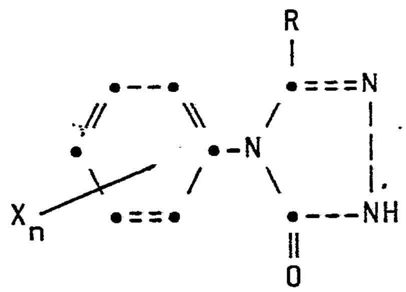

(II)

| X | R | Schmp. °C |
|---|---|---|
| H | $-CH_3$ | 149-152 |
| H | $-C_2H_5$ | 128-130 |
| H | $n-C_3H_7$ | 129-130 |
| H | $i-C_3H_7$ | 169-171 |
| H | $-CH_2-O-CH_3$ | 105-107 |
| H | $-CH_2-CH_2-O-CH_3$ | 241-242 |
| H | $-CH(CH_3)CH_2CH_2CH_3$ | 112-114 |
| H | -cyclpropyl | 129-132 |
| H | -cyclohexyl | 202-204 |
| 4-F | $-CH_3$ | 221-224 |
| 4-F | $-C_2H_5$ | |
| 4-F | $i-C_3H_7$ | 191-193 |
| 3-Cl | $-CH_3$ | 183-185 |
| 3-Cl | $-C_2H_5$ | 177-179 |
| 3-Cl | $n-C_3H_7$ | 135-137 |
| 3-Cl | $i-C_3H_7$ | 148-151 |
| 3-Cl | $-CH_2-CH_2-OCH_3$ | 250-251 |
| 3-Cl | -cyclopropyl | 150-152 |
| 4-Br | $-CH_3$ | 141-145 |

| X | R | Schmp. °C |
|---|---|---|
| 4-Br | -cyclopropyl | |
| 3-$CH_3O$- | -$CH_3$ | 135-138 |
| 4-$CH_3O$- | -$CH_3$ | 202-204 |
| 4-$CH_3O$- | -$C_2H_5$ | 159-161 |
| 4-$CH_3O$- | i-$C_3H_7$ | 182-184 |
| 4-$CH_3$- | i-$C_3H_7$ | 196-198 |
| 3-$(CH_3)_3C$- | -$CH_3$ | 173-175 |
| 3-Cl, 4-$CH_3O$- | -$CH_3$ | 207-209 |
| 3-Cl, 4-$CH_3O$- | -$C_2H_5$ | |
| 3-Cl, 4-$CH_3O$- | i-$C_3H_7$ | |
| 3-$CF_3$- | -$CH_3$ | 145-148 |
| 3-$CF_3$- | i-$C_3H_7$ | |
| 3-$CF_3$ | -cyclopropyl | 144-145 |
| 2-F | -cyclopropyl | 163-165 |
| 3,5-$Cl_2$ | -$CH_3$ | |
| 3,5-$Cl_2$ | -$C_2H_5$ | |
| 3,5-$Cl_2$ | -i-$C_3H_7$ | |
| 3,5-$Cl_2$ | n-$C_3H_7$ | 162-165 |
| 4-$NO_2$ | i-$C_3H_7$ | |

Als Beispiele für die neuen Wirkstoffe der Formel I seien im einzelnen genannt:

1-Trichlormethyl-sulfenyl-3-methyl-4-phenyl-1,2,4-triazolidin-5-on,
1-Fluordichlormethyl-sulfenyl-3-methyl-4-phenyl-1,2,4-triazolidin-5-on,
1-Trichlormethyl-sulfenyl-3-ethyl-4-phenyl-1,2,4-triazolidin-5-on,
1-Trichlormethyl-sulfenyl-3-propyl-4-phenyl-1,2,4-triazolidin-5-on,
1-Trichlormethylsulfenyl-3-isopropyl-4-phenyl-1,2,4-triazolidin-5-on,
1-Fluordichlormethyl-sulfenyl-3-isopropyl-4-phenyl-1,2,4-triazolidin-5-on,
1-Trichlormethyl-sulfenyl-3-methoxymethyl-4-phenyl-1,2,4-triazolidin-5-on,
1-Trichlormethyl-sulfenyl-3-(2-methoxyethyl)-4-phenyl-1,2,4-triazolidin-5-on,
1-Fluordichlormethyl-sulfenyl-3-(2-methoxyethyl)-4-phenyl-1,2,4-triazolidin-5-on,
1-Trichlormethyl-sulfenyl-3-cyclopropyl-4-phenyl-1,2,4-triazolidin-5-on,
1-Fluordichlormethyl-sulfenyl-3-cyclopropyl-4-phenyl-1,2,4-triazolidin-5-on,
1-Trichlormethyl-sulfenyl-3-(2-pentyl)-4-phenyl-1,2,4-triazolidin-5-on,
1-Fluordichlormethyl-sulfenyl-3-(2-pentyl)-4-phenyl-1,2,4-triazolidin-5-on,
1-Trichlormethyl-sulfenyl-3-cyclohexyl-4-phenyl-1,2,4-triazolidin-5-on,
1-Fluordichlormethyl-sulfenyl-3-cyclohexyl-4-phenyl-1,2,4-triazolidin-5-on,
1-Trichlormethyl-sulfenyl-3-methyl-4-(4-fluorphenyl)-1,2,4-triazolidin-5-on,

1-Trichlormethyl-sulfenyl-3-ethyl-4-(4-fluorphenyl)-1,2,4-triazolidin-5-on,
1-Fluordichlormethyl-sulfenyl-3-methyl-4-(4-fluorphenyl)-1,2,4-triazolidin-5-on,
1-Fluordichlormethyl-sulfenyl-3-ethyl-4-(4-fluorphenyl)-1,2,4-triazolidin-5-on,
1-Trichlormethyl-sulfenyl-3-isopropyl-4-(4-fluorphenyl)-1,2,4-triazolidin-5-on,
1-Trichlormethyl-sulfenyl-3-methoxymethyl-4-(4-fluorphenyl)-1,2,4-triazolidin-5-on,
1-Fluordichlormethyl-sulfenyl-3-isopropyl-4-(4-fluorphenyl)-1,2,4-triazolidin-5-on,
1-Fluordichlormethyl-sulfenyl-3-methoxymethyl-4-(4-fluorphenyl)-1,2,4-triazolidin-5-on,
1-Trichlormethyl-sulfenyl-3-cyclopropyl-4-(4-fluorphenyl)-1,2,4-triazolidin-5-on,
1-Fluordichlormethyl-sulfenyl-3-cyclopropyl-4-(4-fluorphenyl)-1,2,4-triazolidin-5-on,
1-Trichlormethyl-sulfenyl-3-cyclohexyl-4-(4-fluorphenyl)-1,2,4-triazolidin-5-on,
1-Fluordichlormethyl-sulfenyl-3-cyclohexyl-4-(4-fluorphenyl)-1,2,4-triazolidin-5-on,
1-Trichlormethyl-sulfenyl-3-(2-methoxyethyl)-4-(4-fluorphenyl)-1,2,4-triazolidin-5-on,
1-Fluordichlormethyl-sulfenyl-3-cyclopropyl-4-(4-fluorphenyl)-1,2,4-triazolidin-5-on,
1-Trichlormethyl-sulfenyl-3-methyl-4-(4-chlorphenyl)-1,2,4-triazolidin-5-on,
1-Trichlormethyl-sulfenyl-3-ethyl-4-(4-chlorphenyl)-1,2,4-triazolidin-5-on,
1-Fluordichlormethyl-sulfenyl-3-methyl-4-(4-chlorphenyl)-1,2,4-triazolidin-5-on,
1-Fluordichlormethyl-sulfenyl-3-ethyl-4-(4-chlorphenyl)-1,2,4-triazolidin-5-on,

1-Trichlormethyl-sulfenyl-3-isopropyl-4-(4-chlorphenyl)-1,2,4-triazolidin-5-on,
1-Fluordichlormethyl-sulfenyl-3-isopropyl-4-(4-chlorphenyl)-1,2,4-triazolidin-5-on,
1-Trichlormethyl-sulfenyl-3-methyl-4-(3-chlorphenyl)-1,2,4-triazolidin-5-on,
1-Fluordichlormethyl-sulfenyl-3-methyl-4-(4-chlorphenyl)-1,2,4-triazolidin-5-on,
1-Fluordichlormethyl-sulfenyl-3-methyl-4-(3-chlorphenyl)-1,2,4-triazolidin-5-on,
1-Trichlormethyl-sulfenyl-3-ethyl-4-(3-chlorphenyl)-1,2,4-triazolidin-5-on,
1-Fluordichlormethyl-sulfenyl-3-ethyl-4-(3-chlorphenyl)-1,2,4-triazolidin-5-on,
1-Trichlormethyl-sulfenyl-3-propyl-4-(3-chlorphenyl)-1,2,4-triazolidin-5-on,
1-Trichlormethyl-sulfenyl-3-isopropyl-4-(3-chlorphenyl)-1,2,4-triazolidin-5-on,
1-Fluordichlormethyl-sulfenyl-3-isopropyl-4-(3-chlorphenyl)-1,2,4-triazolidin-5-on,
1-Trichlormethyl-sulfenyl-3-methoxymethyl-4-(3-chlorphenyl)-1,2,4-triazolidin-5-on,
1-Trichlormethyl-sulfenyl-3-(2-methoxyethyl)-4-(3-chlorphenyl)-1,2,4-triazolidin-5-on,
1-Fluordichlormethyl-sulfenyl-3-(2-methoxyethyl)-4-(3-chlorphenyl)-1,2,4-triazolidin-5-on,
1-Trichlormethyl-sulfenyl-3-cyclopropyl-4-(3-chlorphenyl)-1,2,4-triazolidin-5-on,
1-Fluordichlormethyl-sulfenyl-3-cyclopropyl-4-(3-chlorphenyl)-1,2,4-triazolidin-5-on,
1-Trichlormethyl-sulfenyl-3-cyclohexyl-4-(3-chlorphenyl)-1,2,4-triazolidin-5-on,
1-Trichlormethyl-sulfenyl-3-methyl-4-(2-fluorphenyl)-1,2,4-triazolidin-5-on,

1-Fluordichlormethyl-sulfenyl-3-methyl-4-(2-fluorphenyl)-1,2,4-triazolidin-5-on,
1-Trichlormethyl-sulfenyl-3-methyl-4-(2-fluorphenyl)-1,2,4-triazolidin-5-on,
1-Trichlormethyl-sulfenyl-3-isopropyl-4-(2-fluorphenyl)-1,2,4-triazolidin-5-on,
1-Fluordichlormethyl-sulfenyl-3-isopropyl-4-(2-fluorphenyl)-1,2,4-triazolidin-5-on,
1-Trichlormethyl-sulfenyl-3-cyclopropyl-4-(2-fluorphenyl)-1,2,4-triazolidin-5-on,
1-Fluordichlormethyl-sulfenyl-3-cyclopropyl-4-(2-fluorphenyl)-1,2,4-triazolidin-5-on,
1-Trichlormethyl-sulfenyl-3-methyl-4-(4-bromphenyl)-1,2,4-triazolidin-5-on,
1-Fluordichlormethyl-sulfenyl-3-methyl-4-(4-bromphenyl)-1,2,4-triazolidin-5-on,
1-Trichlormethyl-sulfenyl-3-isopropyl-4-(4-bromphenyl)-1,2,4-triazolidin-5-on,
1-Fluordichlormethyl-sulfenyl-3-isopropyl-4-(4-bromphenyl)-1,2,4-triazolidin-5-on,
1-Trichlormethyl-sulfenyl-3-cyclopropyl-4-(4-bromphenyl)-1,2,4-triazolidin-5-on,
1-Fluordichlormethyl-sulfenyl-3-cyclopropyl-4-(4-bromphenyl-1,2,4-triazolidin-5-on,
1-Fluordichlormethyl-sulfenyl-3-methyl-4-(3,4-dichlorphenyl)-1,2,4-triazolidin-5-on,
1-Trichlormethyl-sulfenyl-3-isopropyl-4-(3,4-dichlorphenyl)-1,2,4-triazolidin-5-on,
1-Trichlormethyl-sulfenyl-3-tert.-butyl-4-(3,4-dichlorphenyl)-1,2,4-triazolidin-5-on,
1-Trichlormethyl-sulfenyl-3-methyl-4-(3,5-dichlorphenyl)-1,2,4-triazolidin-5-on,
1-Fluordichlormethyl-sulfenyl-3-methyl-4-(3,5-dichlorphenyl)-1,2,4-triazolidin-5-on,

1-Trichlormethyl-sulfenyl-3-ethyl-4-(3,5-dichlorphenyl)-1,2,4-triazolidin-5-on,
1-Fluordichlormethyl-sulfenyl-3-ethyl-4-(3,5-dichlorphenyl)-1,2,4-triazolidin-5-on,
1-Trichlormethyl-sulfenyl-3-propyl-4-(3,5-dichlorphenyl)-1,2,4-triazolidin-5-on,
1-Fluordichlormethyl-sulfenyl-3-propyl-4-(3,5-dichlorphenyl)-1,2,4-triazolidin-5-on,
1-Trichlormethyl-sulfenyl-3-isopropyl-4-(3,5-dichlorphenyl)-1,2,4-triazolidin-5-on,
1-Fluordichlormethyl-sulfenyl-3-isopropyl-4-(3,5-dichlorphenyl)-1,2,4-triazolidin-5-on,
1-Trichlormethyl-sulfenyl-3-methoxymethyl-4-(3,5-dichlorphenyl)-1,2,4-triazolidin-5-on,
1-Trichlormethyl-sulfenyl-3-isobutyl-4-(3,5-dichlorphenyl)-1,2,4-triazolidin-5-on,
1-Trichlormethyl-sulfenyl-3-methyl-4-(3-methoxyphenyl)-1,2,4-triazolidin-5-on,
1-Fluordichlormethyl-sulfenyl-3-methyl-4-(3-methoxyphenyl)-1,2,4-triazolidin-5-on,
1-Trichlormethyl-sulfenyl-3-isopropyl-4-(3-methoxyphenyl)-1,2,4-triazolidin-5-on,
1-Trichlormethyl-sulfenyl-3-methyl-4-(4-methoxyphenyl)-1,2,4-tri- azolidin-5-on,
1-Fluordichlormethyl-sulfenyl-3-methyl-(4-methoxyphenyl)-1,2,4-triazolidin-5-on,
1-Trichlormethyl-sulfenyl-3-ethyl-4-(4-methoxyphenyl)-1,2,4-triazolidin-5-on,
1-Fluordichlormethyl-sulfenyl-3-ethyl-4-(4-methoxyphenyl)-1,2,4-triazolidin-5-on,
1-Trichlormethyl-sulfenyl-3-isopropyl-4-(4-methoxyphenyl)-1,2,4-triazolidin-5-on,
1-Trichlormethyl-sulfenyl-3-isobutyl-4-(4-methoxyphenyl)-1,2,4-triazolidin-5-on,

1-Fluordichlormethyl-sulfenyl-3-isopropyl-4-(4-methoxyphenyl)-1,2,4-triazolidin-5-on,
1-Fluordichlormethyl-sulfenyl-3-isobutyl-4-(4-methoxyphenyl)-1,2,4-triazolidin-5-on,
1-Trichlormethyl-sulfenyl-3-cyclopropyl-4-(4-methoxyphenyl)-1,2,4-triazolidin-5-on,
1-Trichlormethyl-sulfenyl-3-methyl-4-(4-ethoxyphenyl)-1,2,4-triazolidin-5-on,
1-Trichlormethyl-sulfenyl-3-isopropyl-4-(4-ethoxyphenyl)-1,2,4-triazolidin-5-on,
1-Fluordichlormethyl-sulfenyl-3-isopropyl-4-(4-ethoxyphenyl)-1,2,4-triazolidin-5-on,
1-Trichlormethyl-sulfenyl-3-methyl-4-(3-chlor-4-methoxyphenyl)-1,2,4-triazolidin-5-on,
1-Fluordichlormethyl-sulfenyl-3-methyl-4-(3-chlor-4-methoxyphenyl)-1,2,4-triazolidin-5-on,
1-Trichlormethyl-sulfenyl-3-ethyl-4-(3-chlor-4-methoxyphenyl)-1,2,4-triazolidin-5-on,
1-Fluordichlormethyl-sulfenyl-3-ethyl-4-(3-chlor-4-methoxyphenyl)-1,2,4-triazolidin-5-on,
1-Trichlormethyl-sulfenyl-3-isopropyl-4-(3-chlor-4-methoxyphenyl)-1,2,4-triazolidin-5-on,
1-Fluordichlormethyl-sulfenyl-3-isopropyl-4-(3-chlor-4-methoxyphenyl)-1,2,4-triazolidin-5-on,
1-Trichlormethyl-sulfenyl-3-cyclopropyl-4-(3-chlor-methoxyphenyl)-1,2,4-triazolidin-5-on,
1-Fluordichlormethyl-sulfenyl-3-cyclopropyl-4-(3-chlor-4-methoxyphenyl)-1,2,4-triazolidin-5-on,
1-Trichlormethyl-sulfenyl-3-isobutyl-4-(3-chlor-4-methoxyphenyl)-1,2,4-triazolidin-5-on,
1-Trichlormethyl-sulfenyl-3-cyclohexyl-4-(3-chlor-4-methoxyphenyl)-1,2,4-triazolidin-5-on,
1-Trichlormethyl-sulfenyl-3-isopropyl-4-(3-chlor-4-ethoxyphenyl)-1,2,4-triazolidin-5-on,

1-Fluordichlormethyl-sulfenyl-3-isopropyl-4-(3-chlor-4--eth-oxyphenyl)-1,2,4-triazolidin-5-on,
1-Trichlormethyl-sulfenyl-3-methyl-4-(4-methylphenyl)--1,2,4-triazolidin-5-on,
1-Trichlormethyl-sulfenyl-3-isopropyl-4-(4-methylphenyl)--1,2,4-triazolidin-5-on,
1-Fluordichlormethyl-sulfenyl-3-isopropyl-4-(4-methylphenyl)-1,2,4-triazolidin-5-on,
1-Trichlormethyl-sulfenyl-3-methyl-4-(3-tert.-butylphenyl)-1,2,4-ttriazolidin-5-on,
1-Trichlormethyl-sulfenyl-3-isopropyl-4-(3-tert.-butylphenyl)-1,2,4-triazolidin-5-on,
1-Fluordichlormethyl-sulfenyl-3-isopropyl-4-(3-tert.-butylphenyl)-1,2,4-triazolidin-5-on,
1-Trichlormethyl-sulfenyl-3-methyl-4-(4-tert.-butylphenyl)--1,2,4-triazolidin-5-on,
1-Trichlormethylsulfenyl-3-isopropyl-4-(4-tert.-butylphenyl)-1,2,4-triazolidin-5-on,
1-Fluordichlormethyl-sulfenyl-3-isopropyl-4-(4-tert.-butylphenyl)-1,2,4-triazolidin-5-on,
1-Trichlormethyl-sulfenyl-3-methyl-4-(4-trifluormethylphenyl)-1,2,4-triazolidin-5-on,
1-Fluordichlormethyl-sulfenyl-3-methyl-4-(4-trifluormethylphenyl)-1,2,4-triazolidin-5-on,
1-Trichlormethyl-sulfenyl-3-methyl-4-(3-trifluormethylphenyl)-1,2,4-triazolidin-5-on,
1-Fluordichlormethyl-sulfenyl-3-methyl-4-(3-trifluormethylphenyl)-1,2,4-triazolidin-5-on,
1-Fluordichlormethyl-sulfenyl-3-isopropyl-4-(3-trifluormethylphenyl)-1,2,4-triazolidin-5-on,
1-Trichlormethyl-sulfenyl-3-cyclopropyl-4-(3-trifluormethylphenyl)-1,2,4-triazolidin-5-on,
1-Fluordichlormethyl-sulfenyl-3-cyclopropyl)-4-(3-trifluormethylphenyl)-1,2,4-triazolidin-5-on,

1-Trichlormethyl-sulfenyl-3-methyl-4-(3,5-dichlor-4--methoxyphenyl)-1,2,4-triazolidin-5-on,
1-Fluordichlormethyl-sulfenyl-3-methyl-4-(3,5-dichlor-4--methoxyphenyl)-1,2,4-triazolidin-5-on,
1-Trichlormethyl-sulfenyl-3-methyl-4-(4-nitrophenyl)--1,2,4-triazolidin-5-on,
1-Trichlormethyl-sulfenyl-3-isopropyl-4-(4-nitrophenyl)--1,2,4-triazolidin-5-on,
1-Trichlormethyl-sulfenyl-3-methyl-4-(4-biphenylyl)-1,2,4--triazolidin-5-on,

Beispiel 1

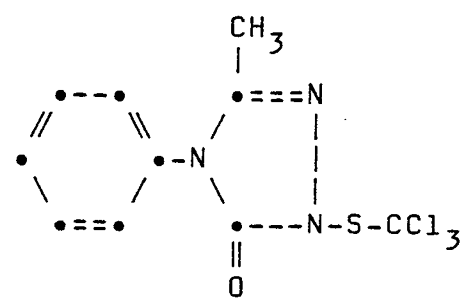

Zu einer Suspension von 17,5 g (0,1 Mol) 3-Methyl-4-phenyl--1,2,4-triazolidin-5-on in 150 ml trockenem Essigester wurden bei etwa 10 bis 15°C nacheinander 19 g (0,102 Mol) Perchlormethylmercaptan und 10 g (0,099 Mol) Triethylamin unter gutem Rühren zugetropft. Nach zweistündigem Rühren bei Raumtemperatur (20°C) wurde das ausgefallene Triethylaminhydrochlorid abgesaugt und mit 40 ml Essigester gewaschen. Das Filtrat wurde zweimal mit je 100 ml Wasser gewaschen (ausgeschüttelt), über $Na_2SO_4$ getrocknet und das Lösungsmittel verdampft (eingeengt). Der Rückstand kristallisierte bei 0°C nach Zugabe von 20 ml Ether. Man erhält 30,15 g (84 % d. Th.) 1-Trichlormethyl-sulfenyl--3-methyl-4-phenyl-1,2,4-triazolidin-5-on als weiße Kristalle vom Schmelzpunkt 142 bis 144°C. (Nr. 1)

Beispiel 2

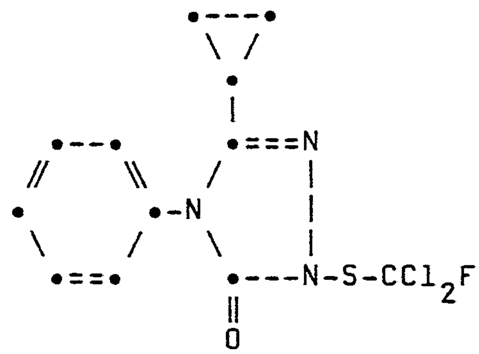

Entsprechend Beispiel 1 wurden 20,1 g (0,1 Mol) 3-Cyclopropyl-4-phenyl-1,2,4-triazolidin-5-on mit 17 g (0,1 Mol) Fluordichlormethyl-sulfenylchlorid umgesetzt. Man erhielt 21,4 g (71 % d.Th.) 1-Fluordichlormethyl-sulfenyl-3-cyclopropyl-4-phenyl-1,2,4-triazolidin-5-on als weiße Kristalle vom Schmelzpunkt 81 bis 83°C. (Nr. 2)

Entsprechend wurden die folgenden Verbindungen der Formel I hergestellt, die durch Ultrarot- und Kernresonanz-Spektroskopie sowie durch Elementaranalyse charakterisiert wurden.

Tabelle 2

| Beispiel Nr. | X | R | Y | IR-Spektrum [KBr][$cm^{-1}$] oder Schmelzpunkt °C |
|---|---|---|---|---|
| 3 | H | $-CH_2-O-CH_3$ | Cl | 85-87 |
| 4 | H | $-C_2H_5$ | Cl | 100-102 |
| 5 | H | $-C_3H_7$-n | Cl | Harz |
| 6 | H | $-C_3H_7$-i | Cl | 116-118 |
| 7 | H | -Cyclopropyl | Cl | 122-123 |
| 8 | H | $-CH_2-CH_2-O-CH_3$ | Cl | 3060, 1730, 1580, 1400, 1200, 1003, 800, 750, |
| 9 | H | $-CH_2-CH_2-O-CH_3$ | F | 3065, 1730, 1578, 1400, 1210, 1040, 810, 759 |
| 10 | H | $-CH(CH_3)CH_2CH_2CH_3$ | Cl | 80-84 |
| 11 | H | $-CH(CH_3)CH_2CH_2-CH_3$ | F | 72-75 |
| 12 | H | -Cyclohexyl | Cl | 117-120 |
| 13 | 4-F | $-CH_3$ | Cl | 145-147 |
| 15 | 4-F | $-C_3H_7$-i | Cl | 105-107 |
| 16 | 2-F | -Cyclopropyl | Cl | 136 |
| 17 | 2-F | -Cyclopropyl | F | 109-110 |
| 18 | 3-Cl | $-CH_3$ | Cl | 120-122 |
| 19 | 3-Cl | $-C_2H_5$ | Cl | 105-107 |
| 20 | 3-Cl | $-C_3H_7$n | Cl | 62-64 |
| 21 | 3-Cl | $-C_3H_7$-i | Cl | 140-142 |

Tabelle 2 (Fortsetzung)

| Beispiel Nr. | X | R | Y | IR-Spektrum [KBr][$cm^{-1}$] oder Schmelzpunkt °C |
|---|---|---|---|---|
| 22 | 3-Cl | $-CH_2CH_2-O-CH_3$ | Cl | 3065, 1730, 1580, 1395, 1205, 1105, 800, 780, 745 |
| 23 | 3-Cl | $-CH_2CH_2-O-CH_3$ | F | 3065, 1730, 1470, 1210, 1105, 1040, 810, 780, 680 |
| 24 | 3-Cl | Cyclopropyl | Cl | 3070, 1730, 1580, 1420, 1210, 1060, 780, 490 |
| 25 | 3-Cl | Cyclopropyl | F | 3070, 1730, 1474, 1418, 1210, 1080, 810, 780, 740 |
| 28 | 4-Br | $-CH_3$ | Cl | 3080, 1735, 1482, 1210, 1112, 1000, 802, 750 |
| 29 | 4-Br | $-CH_3$ | F | 3080, 1735, 1482, 1210, 1112, 1000, 802, 750 |
| 30 | 4-Br | -Cyclopropyl | Cl | 3090, 1735, 1582, 1420, 1210, 990, 810, 750 |
| 31 | 4-Br | -Cyclopropyl | F | 3090, 1730, 1530, 1210, 1065, 989, 808, 740 |
| 32 | $3,5-Cl_2$ | $-C_3H_7-n$ | Cl | 71-73 |
| 33 | $4-CH_3$ | $-C_3H_7-i$ | Cl | 114-116 |
| 34 | $3-C(CH_3)_3$ | $-CH_3$ | Cl | 141-143 |
| 35 | $3-CF_3$ | $-CH_3$ | Cl | 3085, 1735, 1587, 1323, 1210, 1065, 803, 752 |

Tabelle 2 (Fortsetzung)

| Beispiel Nr. | X | R | Y | IR-Spektrum [KBr][$cm^{-1}$] oder Schmelzpunkt °C |
|---|---|---|---|---|
| 36 | 3-$CF_3$ | -$CH_3$ | F | 3080, 1730, 1585, 1325, 1170, 1042, 840, 810 |
| 37 | 3-$CF_3$ | -Cyclopropyl | Cl | 92 |
| 38 | 3-$CF_3$ | -Cyclopropyl | F | 55-57 |
| 39 | 3-$CH_3O$- | -$CH_3$ | Cl | 101-102 |
| 40 | 3-$CH_3O$- | -$CH_3$ | F | 3059, 2925, 1730, 1585, 1480 1390, 1230, 1032, 830, 810 728, 678 |
| 41 | 4-$CH_3O$- | -$CH_3$ | Cl | 136-138 |
| 42 | 4-$CH_3O$- | -$C_2H_5$ | Cl | 128-130 |
| 43 | 4-$CH_3O$- | -$C_3H_7$-i | Cl | 99-101 |
| 44 | 4-$CH_3O$- | -$C_3H_7$-i | F | 81-82 |
| 45 | 3-Cl, 4-$CH_3O$- | -$CH_3$ | Cl | 156-158 |
| 46 | 4-F | -$C_3H_7$-i | F | 78-80 |
| 47 | 3-Cl, 4-$CH_3O$ | -$C_3H_7$-i | F | 120-121 |
| 48 | 3-Cl, 4-$CH_3O$ | -$C_3H_7$-i | Cl | 148-150 |
| 49 | 3-Cl, 4-$CH_3O$ | -$C_2H_5$ | F | 102-103 |
| 50 | 3-Cl, 4-$CH_3O$ | -Cyclohexyl | F | 68-70 |
| 51 | 3-Cl, 4-$CH_3O$ | -Cyclohexyl | Cl | 124-126 |

Tabelle 2 (Fortsetzung)

| Beispiel Nr. | X | R | Y | IR-Spektrum [KBr][$cm^{-1}$] oder Schmelzpunkt °C |
|---|---|---|---|---|
| 52 | 3-Cl, 4-$CH_3O$ | $-CH_3$ | F | 96-97 |
| 53 | 3-Cl, 4-$CH_3O$ | $-C_2H_5$ | Cl | 108-110 |
| 54 | 3-Cl, 4-$CH_3O$ | $-CH_2-O-CH_3$ | F | 98-100 |
| 55 | 3-Cl, 4-$CH_3O$ | $-CH_2-O-CH_3$ | Cl | 130-132 |

Nach entsprechenden Verfahren können die folgenden Verbindungen hergestellt werden.

| Beispiel Nr. | X | R | Y | IR-Spektrum [KBr][$cm^{-1}$] oder Schmelzpunkt °C |
|---|---|---|---|---|
| 14 | 4-F | $-CH_3$ | F | |
| 26 | 4-Cl | $-CH_3$ | Cl | |
| 27 | 4-Cl | $-CH_3$ | F | |

Die neuen Verbindungen zeichnen sich durch eine hervorragende Wirksamkeit gegen ein breites Spektrum von pflanzenpathogenen Pilzen aus. Sie können als Blatt- und Bodenfungizide eingesetzt werden.

Die neuen Verbindungen sind insbesondere geeignet zur Bekämpfung folgender Pflanzenkrankheiten:

Phytophthora infestans an Tomaten und Kartoffeln,
Phytophthora parasitica an Erdbeeren,
Phytophthora cactorum an Äpfeln,
Pseudoperonospora cubensis an Gurken,
Pseudoperonospora humuli an Hopfen,
Peronospora destructor an Zwiebeln,
Peronospora sparsa an Rosen,
Peronospora tabacina an Tabak,
Plasmopara viticola an Reben,
Plasmopara halstedii an Sonnenblumen,
Pythium ultimum an Erbsenkeimlingen,
Botrytis cinerea an Reben, Erdbeeren und Paprika,
Septoria nodorum an Getreide,
Venturia inaequalis (Schorf) an Apfelbäumen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate, Die Anwendungsformen richten sich ganz nach den Verwendungszwecken; sie sollten in jedem Fall eine feine und gleichmäßige Verteilung der wirksamen Substanz gewährleisten. Die Formulierungen werden

in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,1 und 3 kg Wirkstoff oder mehr je ha. Die neuen Verbindungen können auch in Materialschutz eingesetzt werden. Bei der Anwendung der Wirkstoffe im Materialschutz, z.B. als Fungizide für Anstrichfarben und Weich-Polyvinylchlorid, betragen die Aufwandmengen 0,05 bis 5 % (Gew.%) Wirkstoff, bezogen auf das Gesamtgewicht der zu konservierenden Farben bzw. des mikrozid auszurüstenden Polyvinylchlorids. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß

man das Holz mit diesen Mitteln behandelt, beispielsweise tränkt oder anstreicht.

Die Mittel bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile der Verbindung des Beispiels 1 mit 10 Gewichtsteilen N-Methyl-alpha--pyrrolidon und erhält eine Lösung, die zur Anwendung in Form kleinster Tropfen geeignet ist.

II. 10 Gewichtsteile der Verbindung des Beispiels 3 werden in einer Mischung gelöst, die aus 90 Gewichtsteilen Xylol, 6 Gewichtsteilen des Anlagerungsproduktes von 8 bis 10 Mol Ölsäure-N-monoethanolamid, 2 Gewichtsteilen Calciumsalz der Dodecylbenzolsulfonsäure und 2 Gewichtsteilen des Anlagerungsproduktes von 40 Mol Ethylenoxid an 1 Mol Ricinusöl besteht.

III. 20 Gewichtsteile der Verbindung des Beispiels 4 werden in einer Mischung gelöst, die aus 60 Gewichtsteilen Cyclohexanon, 30 Gewichtsteilen Isobutanol, 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gewichtsteile der Verbindung des Beispiels 6 werden in einer Mischung gelöst, die aus 25 Gewichtsteilen Cyclohexanon, 65 Gewichtsteilen einer Mineral-

ölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gewichtsteile der Verbindung des Beispiels 10 werden mit 3 Gewichtsteilen des Natriumsalzes der Diisobutylnaphthalin-α-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer Hammermühle vermahlen. Durch feines Verteilen der Mischung in Wasser erhält man eine Spritzbrühe.

VI. 3 Gewichtsteile der Verbindung des Beispiels 11 werden mit 97 Gewichtsteilen feinteiligem Kaolin innig vermischt. Man erhält auf diese Weise eine Stäubemittel, das 3 Gew.% des Wirkstoffs enthält.

VII. 30 Gewichtsteile der Verbindung des Beispiels 12 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung des Beispiels 13 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit Wasser erhält man eine wäßrige Dispersion.

IX. 20 Teile der Verbindung des Beispiels 17 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den neuen Verbindungen kombiniert werden können, sind beispielsweise:

Schwefel,
Dithiocarbamate und deren Derivate, wie
Ferridimethyldithiocarbamat,
Zinkdimethyldithiocarbamat,
Mangan-Zink-ethylendiamin-bis-dithiocarbamat und

Zinkethylenbisdithiocarbamat,
Tetramethylthiuramdisulfide,
Ammoniak-Komplex von Zink-(N,N-ethylen-bis-dithiocarbamat) und N,N'-Polyethylen-bis-(thiocarbamoyl)-disulfid,
Zink-(N,N'-propylen-bis-dithiocarbamat),

Ammoniak-Komplex von Zink-(N,N'-propylen-bis-dithiocarbamat) und N,N'-Polypropylen-bis-(thiocarbamoyl)-disulfid;

Nitroderivate, wie
Dinitro-(1-methylheptyl)-phenylcrotonat,
2-sec.-Butyl-4,6-dinitrophenyl-3,3-dimethylacrylat,
2-sec.-Butyl-4,6-dinitrophenyl-isopropylcarbonat;

heterocyclische Substanzen, wie
N-(1,1,2,2-Tetrachlorethylthio)-tetrahydrophthalimid,
N-Trichlormethylthio-tetrahydrophthalimid,
2-Heptadecyl-2-imidazolin-acetat
2,4-Dichlor-6-(o-chloranilino)-s-triazin
O,O-Diäthyl-phthalimidophonothioat
5-Amino-1-(bis-(dimethylamino)-phosphinyl)-3-phenyl-1,2,4-triazol)
2,3-Dicyano-1,4-Dithioaanthrachinon
2-Thio-1,3-dithio-(4,5-b)-chinoxalin
1-(Butylcarbamoyl)-2-benzimidazol-carbaminsäuremethylester
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin-4,4-dioxid
2,3-Dihydro-5-carboxanilido-6-methyl-1,4-oxathiin
2-(Furyl-(2))-benzimidazol
N,N'-[1,4-Piperazindiyl-bis-(2,2,2-trichlor-äthyliden)]-bis-formamid
2-(Thiazolyl-(4)-benzimidazol
5-Butyl-2-dimethylamino-4-hydroxy-6-methyl-pyrimidin
Bis-(p-chlorphenyl)-3-pyridinmethanol
1,2-Bis-(3-äthoxycarbonyl-2-thioureido)-benzol
1,2-Bis-(3-methoxycarbonyl-2-thioureido)-benzol
sowie verschiedene Fungizide, wie

Dodecylguanidinacetat
3-(3-(3,5-Dimethyl-2-oxycyclohexyl)-2-hydroxyäthyl)-glutarimid
Hexachlorbenzol
N-Dichlorfluormethylthio-N',N'-dimethyl-N-phenyl-schwefelsäurediamid
2,5-Dimethyl-furan-3-carbonsäureanilid
2-Methyl-benzoesäure-anilid
2-Jod-benzoesäure-anilid
1-(3,4-Dichloranilino)-1-formylamino-2,2,2-trichloräthan
2,6-Dimethyl-N-tridecyl-morpholin bzw. dessen Salze
2,6-Dimethyl-N-cyclododecyl-morpholin bzw. dessen Salze
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
α-(2-Chlorphenyl)-α-(4-chlorphenyl)-5-pyrimidin-methanol
Organozinnverbindungen, wie Tributylzinnoxid und Tributylzinnbenzoat
Methylenbisthiocyanat
Alkyl-dimethyl-benzylammoniumchlorid
Cetyl-pyridiniumchlorid
chlorierte Phenole, wie Tetra- und Pentachlorphenol
Tetrachlorisophthalsäuredinitril
N-Cyclohexyl-N-methoxy-2,5-dimethyl-furan-3-carbonsäureamid
2,4,5-Trimethyl-furan-carbonsäureanilid
N-Phenyl-N,N'-dimethyl-N'-fluordichlormethyl-thiosulfonyl-diamid
2-Thiocyanomethyl-thiobenzothiazol
Mercaptobenzthiazol
Kupfernaphthenat
Alkali- und Metallsalze des N'-Hydroxy-N-cyclohexyl-diazeniumoxids

p-Chlorphenyl-3-propargyl-format
3-Jod-2-propynyl-butyl-carbamat.

Für die folgenden Versuche wurden die folgenden Wirkstoffe als Vergleichsmittel verwendet:

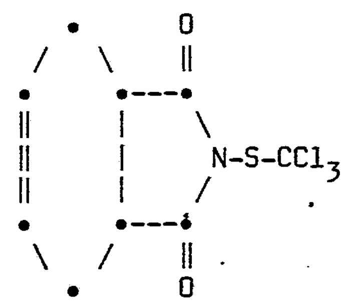

Vergleichsmittel A

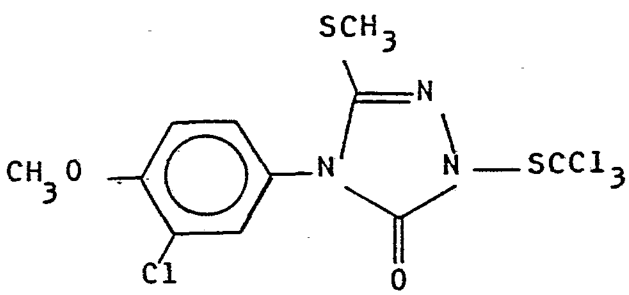

Vergleichsmittel B

Versuch 1

Wirksamkeit gegen Botrytis cinerea an Paprika

Paprikasämlinge der Sorte "Neusiedler Ideal Elite" wurden, nachdem sich 4 bis 5 Blätter gut entwickelt hatten, mit wäßrigen Suspensionen, die 80 % Wirkstoff und 20 % Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hatte sich die Krankheit auf den unbehandelten Kontrollpflanzen so stark entwickelt,

daß die entstandenen Blattnekrosen den überwiegenden Teil der Blätter bedeckten.

Das Ergebnis des Versuches zeigte, daß beispielsweise die Verbindungen 1, 3, 4, 6, 10, 11, 12, 13, 17, 28, 29, 35, 36 und 45 als 0,05 %ige Wirkstoffbrühe eine bessere fungizide Wirkung (beispielsweise 100 %) hatten als der bekannte Wirkstoff A (beispielsweise 70 %).

<u>Versuch 2</u>

Wirksamkeit gegen Phytophthora infestans an Tomaten

Blätter von Topfpflanzen der Sorte "Große Fleischtomate" wurden mit wäßriger Spritzbrühe, die 80 % Wirkstoff und 20 % Emulgiermittel in der Trockensubstanz enthielt, besprüht. Nach dem Antrocknen des Spritzbelages wurden die Blätter mit einer Zoosporenaufschwemmung des Pilzes Phytophthora infestans infiziert. Die Pflanzen wurden dann in einer wasserdampfgesättigten Kammer bei Temperaturen zwischen 16 und 18°C aufgestellt. Nach 5 Tagen hatte sich die Krankheit auf den unbehandelten, jedoch infizierten Kontrollpflanzen so stark entwickelt, daß die fungizide Wirksamkeit der Substanzen beurteilt werden konnte. Das Ergebnis des Versuches zeigte, daß beispielsweise die Verbindungen 1, 6, 13, 17, 18, 24, 28, 39 und 45 als 0,025 %ige Wirkstoffbrühe eine bessere fungizide Wirkung (beispielsweise 97 %) zeigten als der Wirkstoff A (beispielsweise 60 %).

Versuch 3

Wirksamkeit gegen Septoria nodorum

Blätter von in Töpfen gewaschsenen Weizenkeimlingen der Sorte "Jubilar" wurden mit wäßriger Spritzbrühe die 80 % Wirkstoff und 20 % Emulgiermittel in der Trockensubstanz enthielt, besprüht, nach dem Antrocknen des Spritzbelages abgeschnitten und in Schalen mit wäßriger Benzimidazollösung (25 ppm) ausgelegt. Dann wurden die Blätter mit einer wäßrigen Sporensuspension von Septoria nodorum infiziert und abgedeckt. Nach 7-tägigem Stehen bei 20 bis 22°C wurde das Ausmaß der Pilzentwicklung ermittelt.

Das Ergebnis des Versuches zeigte, daß beispielsweise die Wirkstoffe 3, 7, 12, 28, 30, 35 und 39 als 0,1 %ige Wirkstoffbrühe eine gute fungizide Wirkung zeigten (beispielsweise 100 %).

Versuch 4

Wirksamkeit gegen holzverfärbende Pilze

Zur Herstellung eines öligen Holzschutzmittels mit 1 % (Gew.%) Wirkstoff wurde zunächst 1 Teil (Gewichtsteil) der Verbindung gemäß Beispiel 2 unter leichtem Erwärmen in 55 Teilen einer aromatenreichen Benzinfraktion gelöst. Anschließend wurden 10 Teile eines Alkylharzes zugefügt und bei Raumtemperatur mit Benzin auf 100 Teile ergänzt. In entsprechender Weise wurden ölige Holzschutzmittel mit 0,25 bis 5 Gew.% Wirkstoff gemäß den Beispielen 2 und 40 hergestellt.

Kiefernsplintholzbrettchen in Abmessungen von 93x45x9 mm wurden in der Mitte des Brettchens mit einer Sägekerbe versehen, so daß zwei quadratische, gleichgroße Flächen entstanden. Die eine Fläche diente als Kontrollfläche, die andere wurde in zwei Arbeitsgängen mit jeweils 100 $g/m^2$ des Holzschutzmittels behandelt. Die unbehandelten Kontrollflächen wurden mit den gleichen Mengen wirkstofffreiem Leinölfirnis behandelt. Nach 1- bis 2-tägiger Wartezeit wurden alle Brettchen oberseits mit einem ölmodifizierten Kunstharzlack auf Alkydharzbasis gestrichen. Drei Tage nach Aufbringung des Lackanstriches wurden die Brettchen an unbeschatteter Stelle im Freien waagrecht auf Holzroste aufgelegt und 6 Monate lang der Witterung ausgesetzt. Nach Beendigung der sechs Monate erfolgte im Labor der Einbau in 500 $cm^3$ fassende Glasschalen und nach Sterilisation der Prüfkörper mit Propylenoxid wurde die künstliche Infektion mit den Pilzen Pullularia pullulans und Sclerophoma pityophila, die in einer 2 % Malzextraktlösung kultiviert wurde, vorgenommen. Die Aufstellung der Glasschalen erfolgte bei 20 bis 23°C und einer Luftfeuchtigkeit von mindestens 75 %. Nach 6 Wochen war der Versuch beendet.

Nach Abschluß des Versuches waren die Oberfläche der unbehandelten Kontrollflächen zu 90 % durch den Einfluß der Pilze Pullularia pullulans und Sclerophoma pityophila blau gefärbt; auch unter der Oberfläche war das Holz blau gefärbt. Die mit den 1 %igen Holzschutzmitteln der Wirkstoffe Beispiele 2 und 40 behandelten Prüfflächen zeigten keine Anzeichen einer Blaufärbung. Darüber hinaus war auch das Innere des Holzes frei von Blaufärbung.

Versuch 5

Wirksamkeit gegen Phytophthora infestans an Tomaten

Blätter von Topfpflanzen der Sorte "Große Fleischtomate" wurden mit wäßriger Spritzbrühe, die 80 % Wirkstoff und 20 % Emulgiermittel in der Trockensubstanz enthielt, besprüht. Nach dem Antrocknen des Spritzbelages wurden die Blätter mit einer Zoosporenaufschwemmung des Pilzes Phytophthora infestans infiziert. Die Pflanzen wurden dann in einer wasserdampfgesättigten Kammer bei Temperaturen zwischen 16 und 18°C aufgestellt. Nach 5 Tagen hatte sich die Krankheit auf den unbehandelten, jedoch infizierten Kontrollpflanzen so stark entwickelt, daß die fungizide Wirksamkeit der Substanzen beurteilt werden konnte.

Das Ergebnis des Versuches zeigte, daß beispielsweise die Verbindung 45 als 0,05; 0,025 oder 0,012 %ige Wirkstoffbrühe eine bessere fungizide Wirkung (beispielsweise 100 %) hatte als der Wirkstoff B (beispielsweise 70 %).

## Patentansprüche

1. 1-(Trihalogenmethyl-sulfenyl)-4-aryl-1,2,4-triazolidin--5-on der allgemeinen Formel

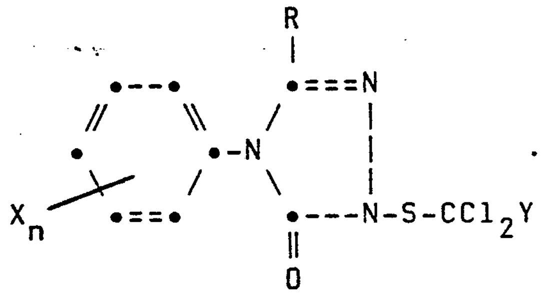

(I)

in der
R einen gegebenenfalls durch Methoxyl oder Ethoxyl substituierten Alkylrest mit 1 bis 6 C-Atomen, oder einen Alkenylrest mit 2 bis 6 C-Atomen oder einen gegebenenfalls durch Methoxyl substituierten Cycloalkylrest mit 3 bis 7 C-Atomen bedeutet,
X Wasserstoff, Halogen, eine gegebenenfalls durch Fluor, Chlor oder Brom substituierte Alkyl- oder Alkoxygruppe mit 1 bis 5 C-Atomen, Alkenyl mit 2 bis 4 C-Atomen, Cyan, Nitro, Phenyl oder Phenoxy bedeutet, wobei der Phenyl- oder der Phenoxyrest durch Fluor, Chlor, Brom oder Alkyl mit 1 bis 4 C-Atomen substituiert sein kann,
n eine ganze Zahl von 1 bis 5 ist und
Y Fluor oder Chlor bedeutet.

2. Fungizid enthaltend ein 1-(Trihalogenmethyl-sulfenyl)-4-aryl-1,2,4-triazolidin-5-on der allgemeinen Formel

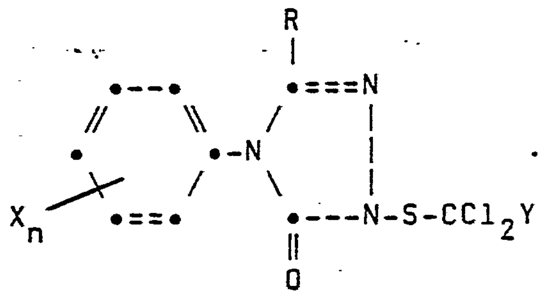

(I)

in der

R einen gegebenenfalls durch Methoxyl oder Ethoxyl substituierten Alkylrest mit 1 bis 6 C-Atomen, oder einen Alkenylrest mit 2 bis 6 C-Atomen oder einen gegebenenfalls durch Methoxyl substituierten Cycloalkylrest mit 3 bis 7 C-Atomen bedeutet,

X Wasserstoff, Halogen, eine gegebenenfalls durch Fluor, Chlor oder Brom substituierte Alkyl- oder Alkoxygruppe mit 1 bis 5 C-Atomen, Alkenyl mit 2 bis 4 C-Atomen, Cyan, Nitro, Phenyl oder Phenoxy bedeutet, wobei der Phenyl- oder der Phenoxyrest durch Fluor, Chlor, Brom oder Alkyl mit 1 bis 4 C-Atomen substituiert sein kann,

n eine ganze Zahl von 1 bis 5 ist und

Y Fluor oder Chlor bedeutet.

3. Fungizid enthaltend einen festen oder flüssigen Trägerstoff und ein 1-(Trihalogenmethyl-sulfenyl)-4-aryl-1,2,4-triazolidin-5-on der allgemeinen Formel

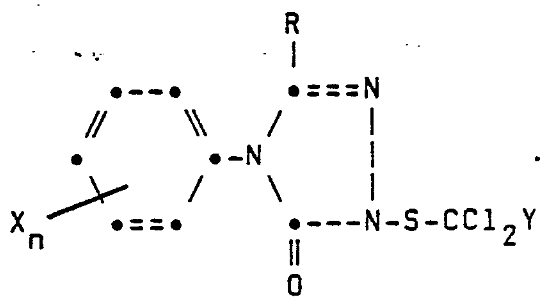

(I)

in der

R einen gegebenenfalls durch Methoxyl oder Ethoxyl substituierten Alkylrest mit 1 bis 6 C-Atomen, oder einen Alkenylrest mit 2 bis 6 C-Atòmen oder einen gegebenenfalls durch Methoxyl substituierten Cycloalkylrest mit 3 bis 7 C-Atomen bedeutet,

X Wasserstoff, Halogen, eine gegebenenfalls durch Fluor, Chlor oder Brom substituierte Alkyl- oder Alkoxygruppe mit 1 bis 5 C-Atomen, Alkenyl mit 2 bis 4 C-Atomen, Cyan, Nitro, Phenyl oder Phenoxy bedeutet, wobei der Phenyl- oder der Phenoxyrest durch Fluor, Chlor, Brom oder Alkyl mit 1 bis 4 C-Atomen substituiert sein kann,

n eine ganze Zahl von 1 bis 5 ist und

Y Fluor oder Chlor bedeutet.

4. Verfahren zur Herstellung eines Fungizids, dadurch gekennzeichnet, daß man einen festen oder flüssigen Trägerstoff vermischt mit einem 1-(Trihalogenmethyl-sulfenyl)-4-aryl-1,2,4-triazolidin-5-on der allgemeinen Formel

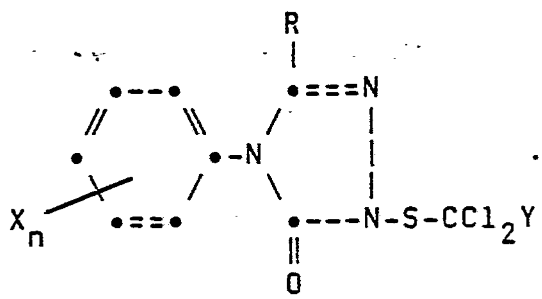

(I)

in der
R einen gegebenenfalls durch Methoxyl oder Ethoxyl substituierten Alkylrest mit 1 bis 6 C-Atomen, oder einen Alkenylrest mit 2 bis 6 C-Atomen oder einen gegebenenfalls durch Methoxyl substituierten Cycloalkylrest mit 3 bis 7 C-Atomen bedeutet,
X Wasserstoff, Halogen, eine gegebenenfalls durch Fluor, Chlor oder Brom substituierte Alkyl- oder Alkoxygruppe mit 1 bis 5 C-Atomen, Alkenyl mit 2 bis 4 C-Atomen, Cyan, Nitro, Phenyl oder Phenoxy bedeutet, wobei der Phenyl- oder der Phenoxyrest durch Fluor, Chlor, Brom oder Alkyl mit 1 bis 4 C-Atomen substituiert sein kann,
n eine ganze Zahl von 1 bis 5 ist und
Y Fluor oder Chlor bedeutet.

5. Verfahren zur Bekämpfung von Pilzen, dadurch gekennzeichnet, daß man die Pilze oder die vor Pilzbefall zu schützenden Gegenstände behandelt mit einem 1-(Trihalogenmethyl-sulfenyl)-4-aryl-1,2,4-triazolidin-5-on der allgemeinen Formel

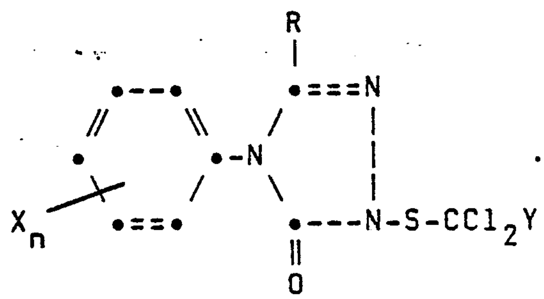

. (I)

in der

R einen gegebenenfalls durch Methoxyl oder Ethoxyl substituierten Alkylrest mit 1 bis 6 C-Atomen, oder einen Alkenylrest mit 2 bis 6 C-Atomen oder einen gegebenenfalls durch Methoxyl substituierten Cycloalkylrest mit 3 bis 7 C-Atomen bedeutet,

X Wasserstoff, Halogen, eine gegebenenfalls durch Fluor, Chlor oder Brom substituierte Alkyl- oder Alkoxygruppe mit 1 bis 5 C-Atomen, Alkenyl mit 2 bis 4 C-Atomen, Cyan, Nitro, Phenyl oder Phenoxy bedeutet, wobei der Phenyl- oder der Phenoxyrest durch Fluor, Chlor, Brom oder Alkyl mit 1 bis 4 C-Atomen substituiert sein kann,

n eine ganze Zahl von 1 bis 5 ist und

Y Fluor oder Chlor bedeutet.

6. Verfahren zur Herstellung eines 1-(Trihalogenmethyl-sulfenyl)-4-aryl-1,2,4-triazolidin-5-on der allgemeinen Formel

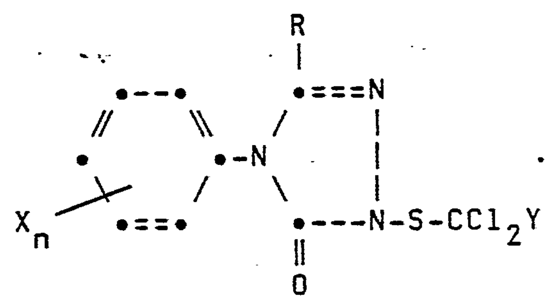

(I)

in der

R einen gegebenenfalls durch Methoxyl oder Ethoxyl substituierten Alkylrest mit 1 bis 6 C-Atomen, oder einen Alkenylrest mit 2 bis 6 C-Atomen oder einen gegebenenfalls durch Methoxyl substituierten Cycloalkylrest mit 3 bis 7 C-Atomen bedeutet,

X Wasserstoff, Halogen, eine gegebenenfalls durch Fluor, Chlor oder Brom substituierte Alkyl- oder Alkoxygruppe mit 1 bis 5 C-Atomen, Alkenyl mit 2 bis 4 C-Atomen, Cyan, Nitro, Phenyl oder Phenoxy bedeutet, wobei der Phenyl- oder der Phenoxyrest durch Fluor, Chlor, Brom oder Alkyl mit 1 bis 4 C-Atomen substituiert sein kann,

n eine ganze Zahl von 1 bis 5 ist und

Y Fluor oder Chlor bedeutet,

<u>dadurch gekennzeichnet</u>, daß man ein 4-Aryl-1,2,4-triazolidin-5-on der Formel

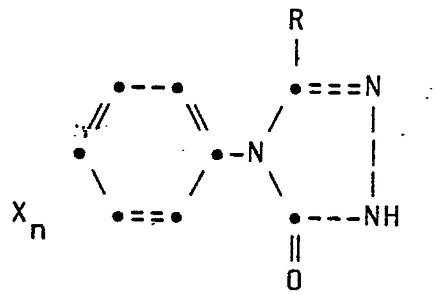

(II)

worin R, X und n die in Anspruch 1 angegebene Bedeutung haben, mit einem Trihalogenmethyl-sulfenylchlorid der Formel

$$YCCl_2\text{-}S\text{-}Cl$$

worin Y die in Anspruch 1 angegebene Bedeutung hat, gegebenenfalls in Gegenwart eines Lösungsmittels, gegebenenfalls unter Zusatz einer anorganischen oder organischen Base bei Temperaturen zwischen -30 und +100°C umsetzt.

7. 1-(Trihalogenmethyl-sulfenyl)-4-aryl-1,2,4-triazolidin-5-on gemäß Anspruch 1, dadurch gekennzeichnet, daß R Methyl oder Ethyl und X Halogen, Methyl oder Methoxy bedeutet.

8. Fungizid enthaltend ein 1-(Trihalogenmethyl-sulfenyl)-4-aryl-1,2,4-triazolidin-5-on definiert wie in Anspruch 1, dadurch gekennzeichnet, daß R Methyl oder Ethyl und X Halogen, Methyl oder Methoxy bedeutet.

Patentansprüche (für Österreich)

1. Fungizid enthaltend ein 1-(Trihalogenmethyl-sulfenyl)-4-aryl-1,2,4-triazolidin-5-on der allgemeinen Formel

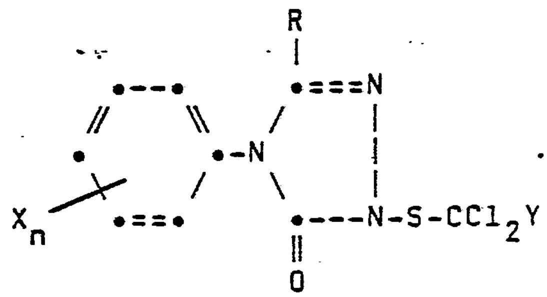

(I)

in der

R einen gegebenenfalls durch Methoxyl oder Ethoxyl substituierten Alkylrest mit 1 bis 6 C-Atomen, oder einen Alkenylrest mit 2 bis 6 C-Atomen oder einen gegebenenfalls durch Methoxyl substituierten Cycloalkylrest mit 3 bis 7 C-Atomen bedeutet,

X Wasserstoff, Halogen, eine gegebenenfalls durch Fluor, Chlor oder Brom substituierte Alkyl- oder Alkoxygruppe mit 1 bis 5 C-Atomen, Alkenyl mit 2 bis 4 C-Atomen, Cyan, Nitro, Phenyl oder Phenoxy bedeutet, wobei der Phenyl- oder der Phenoxyrest durch Fluor, Chlor, Brom oder Alkyl mit 1 bis 4 C-Atomen substituiert sein kann,

n eine ganze Zahl von 1 bis 5 ist und

Y Fluor oder Chlor bedeutet.

2. Fungizid enthaltend einen festen oder flüssigen Trägerstoff und ein 1-(Trihalogenmethyl-sulfenyl)-4-aryl-1,2,4-triazolidin-5-on der allgemeinen Formel

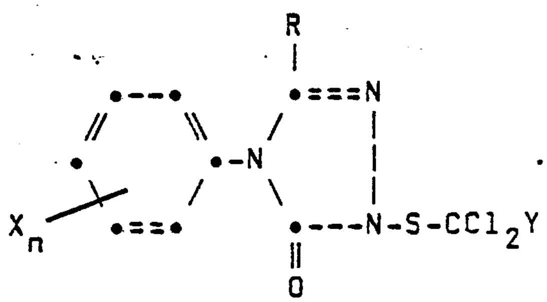

(I)

in der

R einen gegebenenfalls durch Methoxyl oder Ethoxyl substituierten Alkylrest mit 1 bis 6 C-Atomen, oder einen Alkenylrest mit 2 bis 6 C-Atomen oder einen gegebenenfalls durch Methoxyl substituierten Cycloalkylrest mit 3 bis 7 C-Atomen bedeutet,

X Wasserstoff, Halogen, eine gegebenenfalls durch Fluor, Chlor oder Brom substituierte Alkyl- oder Alkoxygruppe mit 1 bis 5 C-Atomen, Alkenyl mit 2 bis 4 C-Atomen, Cyan, Nitro, Phenyl oder Phenoxy bedeutet, wobei der Phenyl- oder der Phenoxyrest durch Fluor, Chlor, Brom oder Alkyl mit 1 bis 4 C-Atomen substituiert sein kann,

n eine ganze Zahl von 1 bis 5 ist und

Y Fluor oder Chlor bedeutet.

3. Verfahren zur Herstellung eines Fungizids, dadurch gekennzeichnet, daß man einen festen oder flüssigen Trägerstoff vermischt mit einem 1-(Trihalogenmethyl-sulfenyl)-4-aryl-1,2,4-triazolidin-5-on der allgemeinen Formel

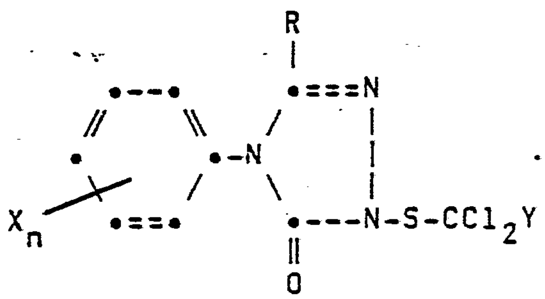

(I)

in der

R einen gegebenenfalls durch Methoxyl oder Ethoxyl substituierten Alkylrest mit 1 bis 6 C-Atomen, oder einen Alkenylrest mit 2 bis 6 C-Atomen oder einen gegebenenfalls durch Methoxyl substituierten Cycloalkylrest mit 3 bis 7 C-Atomen bedeutet,

X Wasserstoff, Halogen, eine gegebenenfalls durch Fluor, Chlor oder Brom substituierte Alkyl- oder Alkoxygruppe mit 1 bis 5 C-Atomen, Alkenyl mit 2 bis 4 C-Atomen, Cyan, Nitro, Phenyl oder Phenoxy bedeutet, wobei der Phenyl- oder der Phenoxyrest durch Fluor, Chlor, Brom oder Alkyl mit 1 bis 4 C-Atomen substituiert sein kann,

n eine ganze Zahl von 1 bis 5 ist und

Y Fluor oder Chlor bedeutet.

4. Verfahren zur Bekämpfung von Pilzen, dadurch gekennzeichnet, daß man die Pilze oder die vor Pilzbefall zu schützenden Gegenstände behandelt mit einem 1-(Trihalogenmethyl-sulfenyl)-4-aryl-1,2,4-triazolidin--5-on der allgemeinen Formel

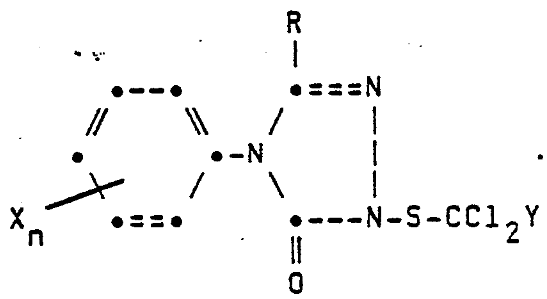

(I)

in der
R einen gegebenenfalls durch Methoxyl oder Ethoxyl substituierten Alkylrest mit 1 bis 6 C-Atomen, oder einen Alkenylrest mit 2 bis 6 C-Atomen oder einen gegebenenfalls durch Methoxyl substituierten Cycloalkylrest mit 3 bis 7 C-Atomen bedeutet,
X Wasserstoff, Halogen, eine gegebenenfalls durch Fluor, Chlor oder Brom substituierte Alkyl- oder Alkoxygruppe mit 1 bis 5 C-Atomen, Alkenyl mit 2 bis 4 C-Atomen, Cyan, Nitro, Phenyl oder Phenoxy bedeutet, wobei der Phenyl- oder der Phenoxyrest durch Fluor, Chlor, Brom oder Alkyl mit 1 bis 4 C-Atomen substituiert sein kann,
n eine ganze Zahl von 1 bis 5 ist und
Y Fluor oder Chlor bedeutet.

5. Verfahren zur Herstellung eines 1-(Trihalogenmethyl-sulfenyl)-4-aryl-1,2,4-triazolidin-5-on der allgemeinen Formel

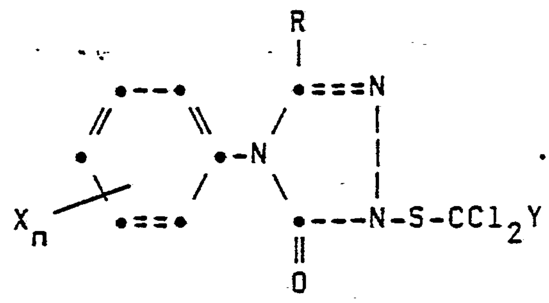 (I)

in der

R einen gegebenenfalls durch Methoxyl oder Ethoxyl substituierten Alkylrest mit 1 bis 6 C-Atomen, oder einen Alkenylrest mit 2 bis 6 C-Atomen oder einen gegebenenfalls durch Methoxyl substituierten Cycloalkylrest mit 3 bis 7 C-Atomen bedeutet,

X Wasserstoff, Halogen, eine gegebenenfalls durch Fluor, Chlor oder Brom substituierte Alkyl- oder Alkoxygruppe mit 1 bis 5 C-Atomen, Alkenyl mit 2 bis 4 C-Atomen, Cyan, Nitro, Phenyl oder Phenoxy bedeutet, wobei der Phenyl- oder der Phenoxyrest durch Fluor, Chlor, Brom oder Alkyl mit 1 bis 4 C-Atomen substituiert sein kann,

n eine ganze Zahl von 1 bis 5 ist und

Y Fluor oder Chlor bedeutet,

dadurch gekennzeichnet, daß man ein 4-Aryl-1,2,4-triazolidin-5-on der Formel

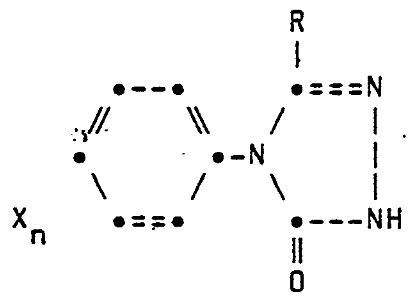

(II)

worin
R, X und n die in Anspruch 1 angegebene Bedeutung haben, mit einem Trihalogenmethyl-sulfenylchlorid der Formel

$$YCCl_2-S-Cl$$

worin
Y die in Anspruch 1 angegebene Bedeutung hat, gegebenenfalls in Gegenwart eines Lösungsmittels, gegebenenfalls unter Zusatz einer anorganischen oder organischen Base bei Temperaturen zwischen -30 und +100°C umsetzt.

6. Fungizid enthaltend ein 1-(Trihalogenmethyl-sulfenyl)-4-aryl-1,2,4-triazolidin-5-on definiert wie in Anspruch 1, dadurch gekennzeichnet, daß R Methyl oder Ethyl und X Halogen, Methyl oder Methoxy bedeutet.

Europäisches Patentamt

# EUROPÄISCHER RECHERCHENBERICHT

0073411 Nummer der Anmeldung

EP 82 10 7506

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
| A,P | --- EP-A-0 039 421 (BASF) * Ansprüche * ----- | 1-6 | C 07 D 249/12<br>A 01 N 43/64 |
| | | | RECHERCHIERTE SACHGEBIETE (Int. Cl. 3) |
| | | | C 07 D 249/00 |

Der vorliegende Recherchenbericht wurde für alle Patentansprüche erstellt.

| Recherchenort | Abschlußdatum der Recherche | Prüfer |
|---|---|---|
| DEN HAAG | 10-12-1982 | CREMERS K. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument